# EUROPEAN PATENT SPECIFICATION

(11) **EP 1 767 232 B1**
(45) Date of publication and mention of the grant of the patent: **03.12.2008**
(21) Application number: 06019529.4
(22) Date of filing: 19.09.2006
(51) Int. Cl.: A61M 1/36, A61M 1/10

(54) **System for monitoring the extracorporeal circulation and the perfusion of medical flows during cardiopulmonary bypass operations**
System zur Überwachung der extrakorporalen Zirkulation und der Infusion von medizinischen Flüssigkeiten während kardiopulmonaler Bypass-Operationen
Système de surveillance de la circulation extracorporelle et de la perfusion des flux médicaux pendant des opérations de pontage cardio-pulmonaire

(30) Priority: 23.09.2005 IT MO20050243
(43) Date of publication of application: 28.03.2007
(73) Proprietor: Rand S.r.l., 41036 Medolla (Modena) (IT)
(72) Inventor: Borra, Mirco, 41019 Soliera (MO) (IT); Bellini, Corrado, 41037 Mirandola (MO) (IT); Galavotti, Daniele, 41037 Mirandola (MO) (IT)
(74) Representative: Feltrinelli, Secondo Andrea

(56) References cited:
- EP-A2- 0 611 228
- EP-A2- 0 998 950
- WO-A2-20/05039671
- DE-A1- 1 501 134
- US-A- 6 071 258
- US-B1- 6 691 047

## Description

The object of the invention is a system for monitoring the extracorporeal circulation and the perfusion of medical flows during cardiopulmonary bypass operations.

Current machines and systems are used for the perfusion of medical fluids and for the monitoring of the relevant therapy, before or during heart surgery with a cardiopulmonary bypass, which conventionally include a support structure in a substantially horizontal form, which is equipped with peristaltic pumps that are associated with a segment of an infusion circuit which is applied, downline, to the patient.

Such machines or systems are generally capable of the administration of medical fluids, such as those used for cardioplegia, that are able to reduce the temperature of the cardiac organ during a surgical operation.

The peristaltic pumps are applied along a segment of the infusion circuit and possess a rotor element equipped with peripheral pressure lobes that can be manually adjusted in relation to the center of rotation by a health professional according to the transverse cross-section of the tube defining the circuit itself.

The use of such machines or systems is generally supported by the use of further equipment such as circuits that are supplied by external pumps and that are able to support the extracorporeal blood circulation of the patient following the bypass of the cardiac organ.

Further equipment such as, for example, pump syringes, are conventionally used for the administration of specific medical fluids. In this case the dosage and the administration intervals are controlled by the health professional who always manually performs these procedures. A perfusion control assembly is known from US 6,071,258 which provides for improved component visibility and accessibility. These current machines or systems present several drawbacks, including the fact that the substantially horizontal form of their support structure implies a larger blood volume in diffused extracorporeal circulation, dimensions that cannot be overlooked inside rooms, such as an operating room, that require a high degree of optimization of the space, and a possible impediment to the movements of the health professionals inside the operating room during a surgical operation.

A further drawback is the fact that the manual calibration of the positions of the lobes of the peristaltic pumps' rotor in relation to the type of pipe used is disadvantageous in terms of the amount of time spent. Furthermore, this implies the need for highly qualified personnel and does not in any case remove the existence of a margin of error due to manual calibrations that are incorrect or even considerably inexact.

The main object of the invention is to eliminate the above-mentioned drawbacks of the current state of the art by developing a system that guarantees reduced dimensions and that guarantees minimal impedance of the health professionals' movements in the operating room.

Another object of the invention is to reduce the volume of blood in extracorporeal circulation.

A further object of the invention is to guarantee the rapid adjustment of the peristaltic pumps' rotors according to the types of tubes used, thereby simultaneously reducing the margins of error during such an operation.

In the context of this technical objective, another object of the invention is to accomplish the preceding objectives with a simple structure that is relatively easy and practical to implement, that is safe to use, and that operates efficiently.

This objective and these goals are all achieved by the present system for the monitoring of the extracorporeal circulation and the perfusion of medical flows during cardiopulmonary bypass operations according to claim 1..

Further features and advantages of the present invention will become more apparent from the detailed description of a preferred, but not exclusive, embodiment of a system for the monitoring of the extracorporeal circulation and the perfusion of medical flows during cardiopulmonary bypass operations, as specified indicatively, but not restrictively, in the combined tables of drawings in which:

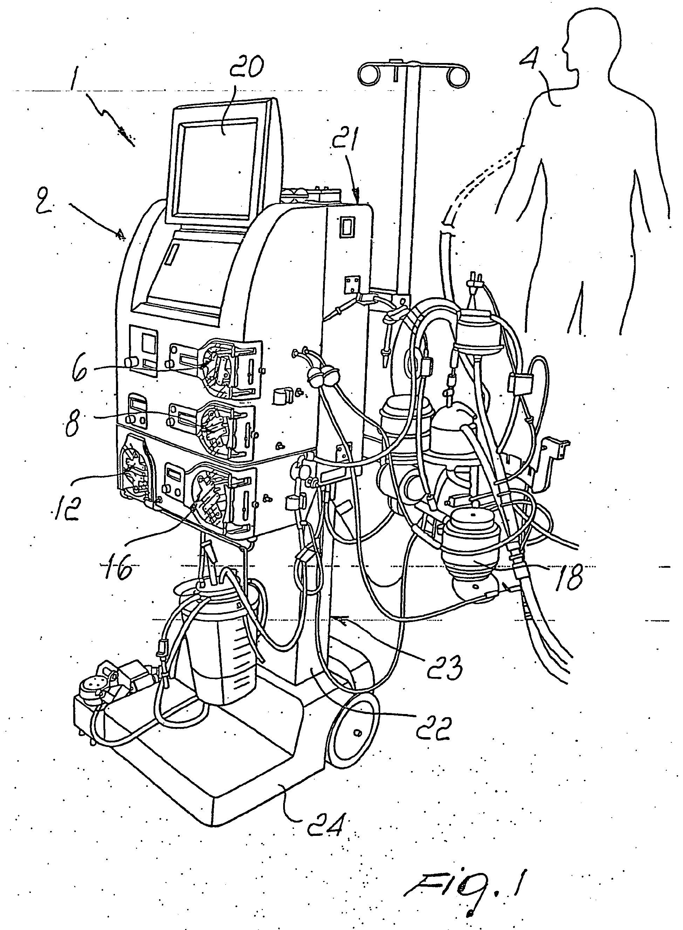

Figure 1 is an axonometric view of the system according to the invention;

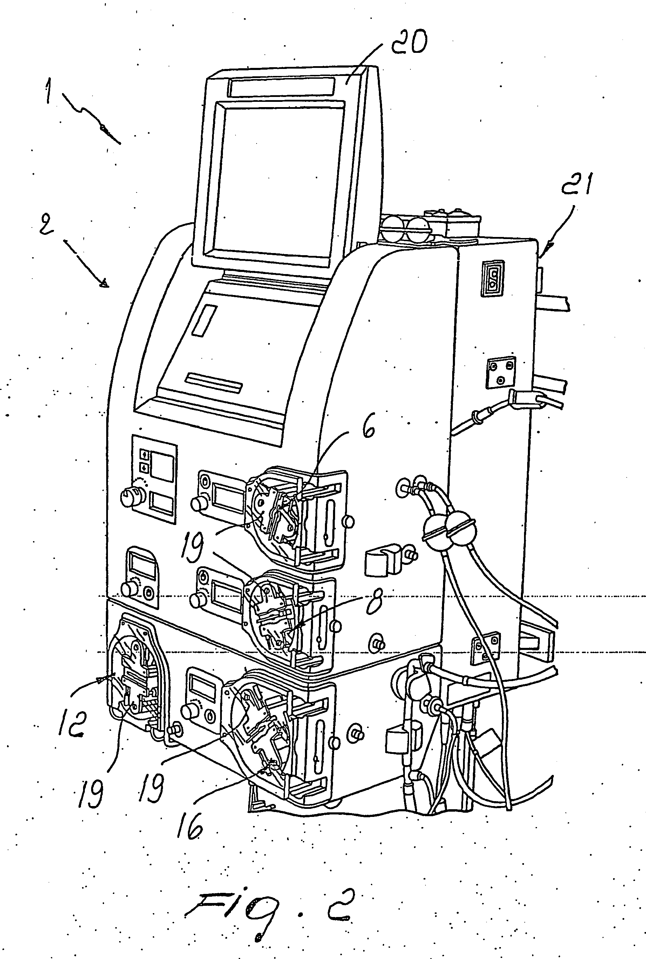

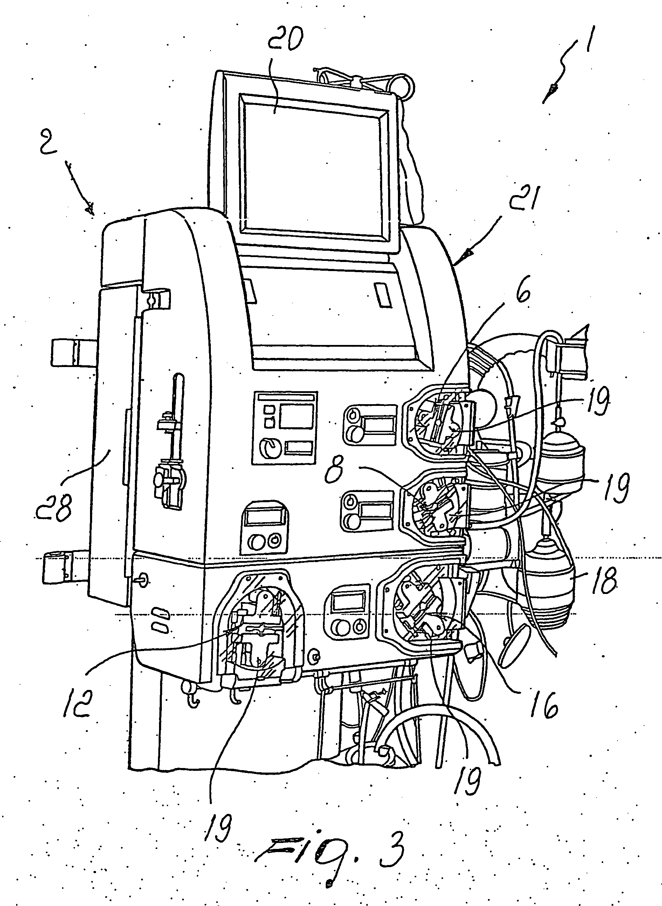

Figures 2 and 3 are axonometric views of a detail of the system according to the invention;

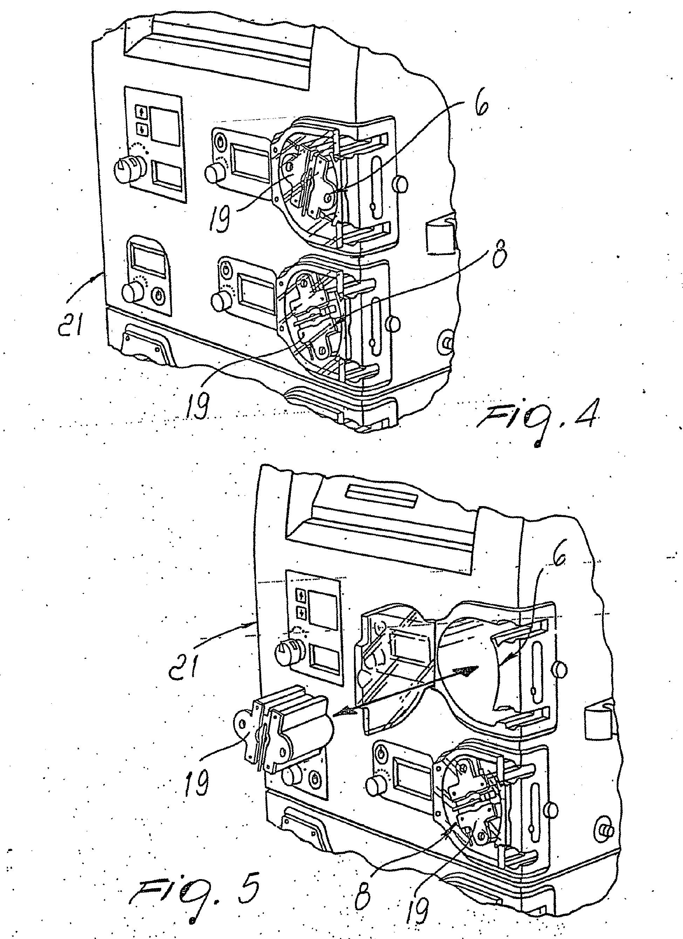

Figures 4 and 5 are views of a detail of the system according to the invention, which illustrates the phases of removing a pump rotor;

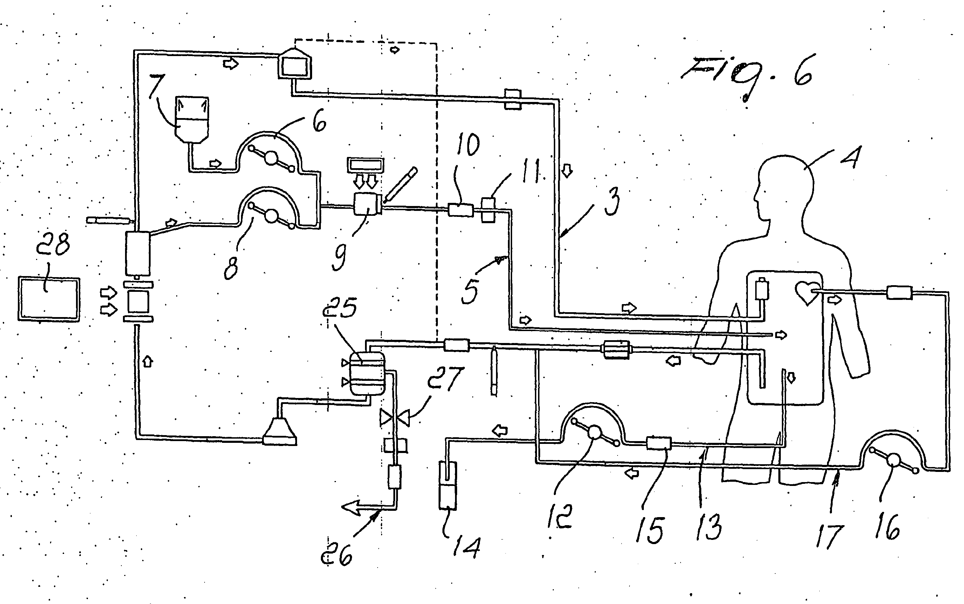

Figure 6 is a general diagram illustrating the functional fluid screen of the system according to the invention.

With special reference to these figures, 1 is used overall to indicate the system for monitoring the extracorporeal circulation and the perfusion of medical flows during cardiopulmonary bypass operations.

The system 1 includes a substantially vertical support structure 2 that can be associated with at least one induced circulation circuit 3 of the blood flow in a patient 4 undergoing a cardiopulmonary bypass operation, and that is equipped with at least one integrated pump that supplies an infusion circuit 5 that infuses a medical fluid to the patient 4.

In detail, a first integrated pump 6 is connected to the inlet of a source of medical fluid 7, specifically, the type used for cardioplegia to cool the patient's heart during a surgical operation, while a second integrated pump 8 is connected to the inlet of the induced circulation circuit 3 for the collection of a predetermined amount of blood from the patient 4.

The blood and the medical fluid collected in this manner are mixed together according to preset amounts (for example, in relation to their respective volumes or weights) and introduced in the infusion circuit 5 connected to the respective outlets of the first and second integrated pump 6 and 8, and are perfused to the patient 4.

Alternatively the first integrated pump 6 can be connected to the inlet of the induced circulation circuit 3 and to the source of medical fluid 7, for the collection of preset amounts of blood and medical fluid, for their mixing, and for the subsequent introduction into the infusion circuit 5.

Furthermore, the infusion circuit 5 can include a cooling device 9 of the blood, a first sensor 10 for measuring the blood pressure, and a second sensor 11 for detecting air bubbles inside the circuit itself.

A third supplementary pump 12 is placed on an aspiration circuit 13 downline from the patient 4 and upline from a collection device 14.

Between the third supplementary pump 12 and the patient is placed a third pressure sensor 15 for measuring the pressure value of the aspirated blood.

The aspiration circuit 13 is used for cleaning the peritoneal zone of the patient 4 of the blood during the surgical operation, and the collection device 14 can recover the collected blood and introduce it into the induced circulation circuit 3.

A fourth supplementary pump 16 is placed on a cardiac ventilation circuit 17 and is associated downline from patient 4 and upline from the induced circulation circuit 3.

Advantageously, both the third supplementary pump 12 and the fourth supplementary pump 16 can be applied to the cardiac ventilation circuit 17 or to the aspiration circuit 13.

Also included is an external supply pump 18 for supplying the induced circulation circuit 3 that is controlled by the means of monitoring and control.

The first and the second integrated pump 6 and 8 are peristaltic type pumps that include, respectively, a rotor element 19 acting on at least one segment of the infusion circuit 5 for the propulsion of the medical fluid toward the patient 4.

The rotor element 19 is associated in a removable manner with a drive body rotated by means of removable coupling means and can be replaced with other rotors 19 that have different rotation diameters according to the dimensions of the transverse cross-section of the tubes that define the induced circulation circuit 3.

In detail, the tubes that are conventionally used in heart surgery have maximum transverse cross-section dimensions of 6.4 mm, 3.2 mm, or 9.5 mm: therefore, for each of the latter can be installed on the integrated pumps 6 and 8 a specific rotor 19 that has an external rotation diameter that is precalibrated and tested by a manufacturer in relation to the exact flow rate that the various tubes must supply, without any need to perform further manual adjustments of the positions of the lobes.

Advantageously, also the first and the second supplementary pump 12 and 16 are peristaltic type pumps that include at least one removable rotor element 19 and that act on, respectively, at least one segment of the aspiration circuit 13 and at least one segment of the ventilation circuit 17.

The means of monitoring and control include a control unit, which is not indicated in the figure for the sake of simplicity, that controls the induced circulation circuit 3 and the infusion circuit 5 and that is equipped with an interface screen 20 used for visual monitoring and for manual operations as well as setting operations, or for verification of volumes or weights, for example by means of a load cell, by a health professional.

In detail, the support structure 2 includes a box body 21 that is supported by a support 22 equipped with means of adjustment of the dimensions; the screen 19 can be folded onto the box body 21 into a closed position. The means of adjustment include a carriage that is integrally associated with said box body and guided, by means of sliding, onto a vertical support 23 that extends from a base 24.

The system 1 also includes means for detecting and removing the air from the induced circulation circuit 3, which includes at least one detecting and signaling device 25 of the presence of air that can be connected to said control unit, and aspiration means of said air that can be activated by said control unit.

In detail, the aspiration means includes a discharge circuit 26 equipped with a valve 27 that can be controlled by the control unit.

The system 1 includes means of heating and cooling the blood inside the induced circulation circuit 3 that include a heating and cooling element 28 that is placed along at least one segment of the induced circulation circuit 3.

The heating and cooling element 28 includes a plurality of Peltier cells, placed next to each other, that can be connected to an electric power supply circuit and a metal sheet that is placed between said plurality of cells and at least one segment of the induced circulation circuit 3.

Usefully, the system 1 includes means for storing the patient's physiological data and the events monitored by said control unit that consist of a CompactFlash type memory card that can be inserted in a connector that is located externally to the box body 21.

Advantageously, the machine 1 also includes an integrated printer that can be controlled by said control unit and that is able to print the values of the physiological data of the patient 4 and the events that occur during the therapy.

Furthermore, the system 1 includes a pump type syringe that can be controlled by the control unit and that can be used for the controlled administration of medical fluids.

In practice it has been verified that the described invention has achieved the proposed objects, and specifically it is underlined that the existence of a support structure with a vertical form guarantees the reduced dimensions of the system and minimal impedance of the health professionals' movements in the operating room.

Furthermore, the possibility of removing and replacing the peristaltic pumps' rotors according to the types of tubes used guarantees the rapidity of those operations for preparing the system for the surgical operation and eliminates the possibility of errors due to the incorrect calibration of the rotor.

The possibility of errors is also reduced spontaneously, and in any case identifiably, due to the existence of means of storage of all the events and the patient's conditions during the operation and the comparison of these data with the desired events and conditions.

The monitoring of the induced circulation circuit and the introduction circuit by means of a single screen, together with the possibility of monitoring and controlling other instruments, such as a pump syringe, makes it possible to limit errors due to confusion by the health professionals, thereby also increasing the speed of the actions taken by the health professionals themselves under emergency conditions.

The invention conceived in this manner is subject to numerous modifications and variations, all of which are comprised in the context of the inventive concept. Furthermore, all the details can be replaced with other elements that are technically equivalent.

In practice the materials used, as well as the corresponding forms and dimensions, can be varied to any degree according to requirements, without this causing said variations to fall outside the purview of the protection of the following claims.

## Claims

1. System (1) for the monitoring of the extracorporeal circulation and the perfusion of medical flows during cardiopulmonary bypass operations including a substantially vertical support structure (2, 21, 22, 23, 24) that can be associated with at least one induced circulation circuit (3) of the blood flow in a patient (4) and that is equipped with at least one integrated pump (6, 8) that supplies at least one infusion circuit (5) that infuses a medical fluid to said patient (4), means of monitoring and control (20) of said induced circulation circuit (3) and said infusion circuit (5) being provided for, wherein said at least one integrated pump (6, 8) is a peristaltic type pump that includes at least one rotor element (19) acting on at least one segment of said infusion circuit (5) for the propulsion of said medical fluid toward said patient (4), and a drive body rotated by said rotor element (19), **characterized in that** the rotor element is precalibrated in relation to the exact flow rate that the various tubes must supply and **in that** the pump includes means of removable coupling of said precalibrated rotor element (19) to said drive body.

2. System according to claim 1, **characterized by** the fact that it comprises a first integrated pump (6) that can be associated with the inlet of said induced circulation circuit (3) and at least one source (7) of said medical fluid and that can be associated with the outlet of said infusion circuit (5).

3. System according to claim 1, **characterized by** the fact that it includes a first integrated pump (6) that can be associated with the inlet of at least one source (7) of said medical fluid and a second integrated pump (8) that can be associated with the inlet of said induced circulation circuit (3), as said first and second integrated pump (6, 8) can be associated with the outlet of said infusion circuit (5) for the mixing of said fluid and of said blood.

4. System according to claim 1, **characterized by** the fact that it includes a third supplementary pump (12) that is placed on a blood aspiration circuit (13) downline from said patient (4) and upline from a collection device (14).

5. System according to claim 1 or 4, **characterized by** the fact that it comprises a fourth supplementary pump (16) that is placed on a cardiac ventilation circuit (17) of said patient (4) and that can be associated downline from said patient (4) and upline from said induced circulation circuit (3).

6. System according to claim 4 or 5, **characterized by** the fact that said third supplementary pump (12) and said fourth supplementary pump (16) are peristaltic type plumps and comprise at least one precalibrated rotor element (19) acting, respectively, on at least one segment of said aspiration circuit (13) and on at least one segment of said ventilation circuit (17), a drive body rotated by said precalibrated rotor element, and means of removable coupling of said precalibrated rotor element (19) to said drive body.

7. System according to claim 1, **characterized by** the fact that said means of monitoring and control comprises a control unit installed on said support structure (2, 21, 22, 23, 24).

8. System according to claim 7, **characterized by** the fact that said control unit can be associated with at least one external pump (18) that supplies said induced circulation circuit (3).

9. System according to claim 1, **characterized by** the fact that it comprises means (25) of detecting and removing the air from said induced circulation circuit (3).

10. System according to claim 7 or 9, **characterized by** the fact that said means of detecting and removing includes at least one device for detecting and signaling (25) the presence of air that can be connected to said control unit, and means of aspiration (26, 27) of said air that can be activated by said control unit.

11. System according to claim 1, **characterized by** the fact that it includes means of heating and cooling (28) the blood contained in said induced circulation circuit (3).

12. System according to claim 11, **characterized by** the fact that said means of heating and cooling include a heating and cooling element (28) that is placed along at least one segment of said induced circulation circuit (3).

13. System according to claim 12, **characterized by** the fact that said heating and cooling element (28) comprises at least one Peltier cell that can be connected to an electric power supply circuit.

14. System according to claim 12 or 13, **characterized by** the fact that said heating and cooling element (28) comprises a plurality of said Peltier cells placed next to each other, with at least one metal sheet placed between said plurality of cells and said at least one segment of said induced circulation circuit (3).

15. System according to claim 1 or 7, **characterized by** the fact that it comprises means of storing the patient's physiological data and the events monitored by said control unit.

16. System according to one or more of the preceding claims, **characterized by** the fact that said means of storing comprises a memory card.

17. System according to claim 1 or 7, **characterized by** the fact that it comprises at least one integrated printer that can be controlled by said control unit.

18. System according to claim 1 or 7, **characterized by** the fact that it comprises a pump type syringe that can be controller by said control unit for the controlled administration of said medical fluids.

19. System according to claim 1, **characterized by** the fact that said support structure comprises a box body (21) that is supported by a support (22) equipped with means of adjustment of the dimensions.

20. System according to claim 19, **characterized by** the fact that said means of adjustment of the dimensions comprises a carriage that is integrally associated with said box body and guided, by means of sliding, onto a vertical support (23) that extends from a base (24).

21. System according to any one of claims 1, 7, 19, **characterized by** the fact that it comprises a screen (20) connected to said control unit, that is contact-activated and that can be folded onto said box body (21) into a closed position.

## Patentansprüche

1. System (1) zur Überwachung der extrakorporalen Zirkulation und der Perfusion medizinischer Flüssigkeitsströme im Verlauf von cardiopulmonären Bypass-Operationen mit einem im Wesentlichen vertikal aufragenden Traggestell (2, 21, 22, 23, 24), das mit mindestens einem in einem Patienten (4) induzierten Blutströmungskreislauf (3) verbindbar ist und mit mindestens einer integrierten Pumpe (6, 8) ausgerüstet ist, welche mindestens einen Infusionskreis (5) versorgt, der eine medizinische Flüssigkeit in den Patienten (4) infundiert, wobei Mittel (20) zur Überwachung und Steuerung des genannten induzierten Blutkreislaufs (3) und des Infusionskreises (5) vorgesehen sind, wobei die mindestens eine genannte integrierte Pumpe (6, 8) als peristaltische Pumpe ausgebildet ist, die mindestens ein Rotorelement (19) enthält, das auf mindestens einen Abschnitt des genannten Infusionskreises (5) für die Förderung der genannten medizinischen Flüssigkeit in Richtung auf den Patienten (4) zu antreibend wirkt, und mit einem Antriebskörper, der mittels des genannten Rotorelements (19) angetrieben wird, **dadurch gekennzeichnet, dass** das Rotorelement in Bezug auf die exakte Durchflussrate, die in den verschiedenen Schläuchen erzielt werden muss, vorkalibriert ist, und dass die Pumpe Mittel zur lösbaren Kupplung des vorkalibrierten Rotorelements (19) mit dem Antriebskörper umfasst.

2. System nach Anspruch 1, **dadurch gekennzeichnet, dass** eine erste integrierte Pumpe vorgesehen ist, die zulaufseitig mit dem genannten induzierten Strömungskreislauf (3) sowie mit mindestens einer Quelle (7) einer medizinischen Flüssigkeit verbindbar ist, und auslaufseitig mit dem genannten Infusionskreis (5) verbindbar ist.

3. System nach Anspruch 1, **dadurch gekennzeichnet, dass** eine erste integrierte Pumpe vorgesehen ist, die einlassseitig mit mindestens einer Quelle (7) für die genannte medizinische Flüssigkeit verbindbar ist und eine zweite integrierte Pumpe (8), die zulaufseitig mit dem genannten induzierten Strömungskreislauf (3) verbindbar ist, wobei die erste und die zweite integrierte Pumpe (6, 8) auslaufseitig mit dem Infusionskreis (5) für die Mischung der genannten Flüssigkeit mit dem Blut verbindbar sind.

4. System nach Anspruch 1, **dadurch gekennzeichnet, dass** es eine dritte Zusatzpumpe (12) umfasst, die in einem Blut-Aspirationskreis (13), stromab von den Patienten (4) und stromauf von einer Sammeleinrichtung (14) angeordnet ist.

5. System nach Anspruch 1 oder Anspruch 4, **dadurch gekennzeichnet, dass** eine vierte Zusatzpumpe (6) vorgesehen ist, die in einem kardialen Sauerstoffaufnahmekreis (17) des Patienten (4) angeordnet und stromab von dem Patienten (4) und stromauf von dem genannten Zirkulationskreis (3) anschließbar ist.

6. System nach Anspruch 4 oder Anspruch 5, **dadurch gekennzeichnet, dass** die genannte dritte Zusatzpumpe (12) und die genannte vierte Hilfspumpe (16) peristaltische Pumpen sind und mindestens ein vorkalibriertes Rotorelement (19) umfassen, das jeweils auf mindestens einen Abschnitt des genannten Aspirationskreises (13) und auf mindestens einen Abschnitt des genannten Sauerstoffaufnahmekreises (17) wirkt, sowie einen Antriebskörper, der mittels des vorkalibrierten Rotorelements rotatorisch antreibbar ist, und Mittel zur lösbaren Verbindung des vorkalibrierten Rotorelements (19) mit dem genannten Antriebskörper.

7. System nach Anspruch 1, **dadurch gekennzeichnet, dass** die genannte Einrichtung zur Überwachung und Kontrolle eine Steuereinheit umfasst, die auf dem genannten Gestell (2, 21, 22, 23, 24) installiert ist.

8. System nach Anspruch 7, **dadurch gekennzeichnet, dass** die Steuereinheit mit mindestens einer externen Pumpe (18) verbindbar ist , die zur Versorgung des induzierten Kreislaufes (3) dient.

9. System nach Anspruch 1, **dadurch gekennzeichnet, dass** es Mittel (25) zum Nachweis und zum Entfernen von Luft aus dem genannten induzierten Kreislauf (3) umfasst.

10. System nach Anspruch 7 oder Anspruch 9, **dadurch gekennzeichnet, dass** die Einrichtung zum Nachweis und zur Abführung von Luft mindestens ein Gerät zum Nachweis und zur Anzeige (25) der Anwesenheit von Luft umfasst, das mit der genannten Steuereinheit verbindbar ist, sowie eine durch die Steuereinheit aktivierbare Einrichtung zum Entfernen (26, 27) der Luft.

11. System nach Anspruch 1, **dadurch gekennzeichnet, dass** Mittel zur Erwärmung und Kühlung (28) des Blutes vorgesehen sind, das in dem erzwungenen Kreislauf enthalten ist.

12. System nach Anspruch 11, **dadurch gekennzeichnet, dass** die Mittel zur Erwärmung und Kühlung mindestens ein Heiz- und Kühlelement (28) umfassen, das längs mindestens eines Abschnitts des induzierten Kreislaufs (3) angeordnet ist.

13. System nach Anspruch 12, **dadurch gekennzeichnet, dass** das Heiz- und Kühlelement (28) mindestens eine Peltierzelle enthält, die mit einer elektrischen Stromversorgung verbindbar ist.

14. System nach Anspruch 12 oder Anspruch 13, **dadurch gekennzeichnet, dass** das Heiz- und Kühlelement (28) eine Mehrzahl von Peltierzellen umfasst, die einander unmittelbar benachbart angeordnet sind, wobei mindestens eine Metallfolie zwischen der genannten Anzahl von Zellen und dem mindestens einen Abschnitt des induzierten Kreislaufes (3) angeordnet ist.

15. System nach Anspruch 1 oder Anspruch 2, **dadurch gekennzeichnet, dass** es Mittel zur Speicherung physiologischer Daten des Patienten sowie der Ereignisse, die mittels der Steuereinheit überwacht werden, umfasst.

16. System nach einem oder mehreren der vorhergehenden Ansprüche, **dadurch gekennzeichnet, dass** die Speichereinrichtung eine Speicherkarte umfasst.

17. System nach Anspruch 1 oder Anspruch 2, **dadurch gekennzeichnet, dass** es mindestens einen integrierten Drucker umfasst, der mittels der Steuereinheit ansteuerbar ist.

18. System nach Anspruch 1 oder Anspruch 7, **dadurch gekennzeichnet, dass** es eine in der Art einer Pumpe ausgebildete Spritze umfasst, die mittels der Steuereinheit zur definierten Verabreichung der genannten medizinischen Flüssigkeiten steuerbar ist.

19. System nach Anspruch 1, **dadurch gekennzeichnet, dass** das Gestell einen Kastenkorpus (21) umfasst, der auf einem Träger (22) steht, der mit Mitteln zur Dimensionsanpassung ausgerüstet ist.

20. System nach Anspruch 19, **dadurch gekennzeichnet, dass** die Mittel zur Anpassung der Dimensionen einen Wagen umfassen, der mit dem genannten Kastenkorpus als Einheit ausgeführt ist und auf einem vertikalen Träger (23), der von einer Basis (24) ausgeht, gleitend verschiebbar ist.

21. System nach einem der Ansprüche 1, 7, 19, **dadurch gekennzeichnet, dass** ein mit der Steuereinheit verbundener Bildschirm (20) vorgesehen ist, der durch Berührung aktivierbar ist und bezüglich des Kastenkorpus (21) in eine geschlossene Position klappbar ist.

## Revendications

1. Système (1) pour le monitorage de la circulation extracorporelle et de la perfusion de flux médicaux pendant des opérations de pontage cardiopulmonaire, comprenant une structure de support sensiblement verticale (2, 21, 22, 23, 24) qui peut être associée à au moins un circuit (3) de circulation induite du flux sanguin d'un patient (4) et qui est équipée d'au moins une pompe intégrée (6, 8) qui alimente au moins un circuit (5) de perfusion qui perfuse un fluide médical audit patient (4), des moyens de monitorage et de commande (20) dudit circuit (3) de circulation induite et dudit circuit (5) de perfusion étant prévus, dans lequel ladite au moins une pompe intégrée (6, 8) est une pompe de type péristaltique qui comprend au moins un élément de rotor (19) agissant sur au moins un segment dudit circuit (5) de perfusion pour la propulsion dudit fluide médical vers ledit patient (4), et un corps d'entraînement entraîné en rotation par ledit élément de rotor (19), ***caractérisé en ce que*** l'élément de rotor est précalibré en relation avec le débit exact que les divers tubes doivent fournir, et ***en ce que*** la pompe comprend des moyens d'accouplement amovible dudit élément de rotor (19) avec ledit corps d'entraînement.

2. Système selon la revendication 1, ***caractérisé par le fait qu**'il* comprend une première pompe intégrée (6) qui peut être associée à l'entrée dudit circuit (3) de circulation induite et à au moins une source (7) dudit fluide médical et qui peut être associée à la sortie dudit circuit (5) de perfusion.

3. Système selon la revendication 1, ***caractérisé par le fait qu**'il* comprend une première pompe intégrée (6) qui peut être associée à l'entrée d'au moins une source (7) dudit fluide médical et une deuxième pompe intégrée (8) qui peut être associée à l'entrée dudit circuit (3) de circulation induite, lesdites première et deuxième pompes intégrées (6, 8) pouvant être associées à la sortie dudit circuit (5) de perfusion pour le mélange dudit fluide et dudit sang.

4. Système selon la revendication 1, ***caractérisé par par le fait qu***'il comprend une troisième pompe supplémentaire (12) qui est placée sur un circuit (13) d'aspiration du sang en aval dudit patient (4) et en amont d'un dispositif de collecte (14).

5. Système selon la revendication 1 ou 4, ***caractérisé par le fait qu**'il* comprend une quatrième pompe supplémentaire (16) qui est placée sur un circuit (17) de ventilation cardiaque dudit patient (4) et qui peut être associée en aval dudit patient (4) et en amont dudit circuit (3) de circulation induite.

6. Système selon la revendication 4 ou 5, ***caractérisé par le fait que*** ladite troisième pompe supplémentaire (12) et ladite quatrième pompe supplémentaire (16) sont des pompes de type péristaltique et comprennent au moins un élément de rotor précalibré (19) agissant respectivement sur au moins un segment dudit circuit d'aspiration (13) et sur au moins un segment dudit circuit de ventilation (17), un corps d'entraînement entraîné en rotation par ledit élément de rotor précalibré, et des moyens d'accouplement amovible dudit élément de rotor précalibré (19) avec ledit corps d'entraînement.

7. Système selon la revendication 1, ***caractérisé par le fait que*** lesdits moyens de monitorage et de commande comprennent une unité de commande installée sur ladite structure de support (2, 21, 22, 23, 24).

8. Système selon la revendication 7, ***caractérisé par le fait que*** ladite unité de commande peut être associée à au moins une pompe extérieure (18) qui alimente ledit circuit (3) de circulation induite.

9. Système selon la revendication 1, ***caractérisé par le fait qu**'il* comprend des moyens (25) de détection et d'élimination de l'air hors dudit circuit (3) de circulation induite.

10. Système selon la revendication 7 ou 9, ***caractérisé par le fait que*** lesdits moyens de détection et d'élimination comprennent au moins un dispositif pour détecter et signaler (25) la présence d'air qui peut être raccordé à ladite unité de commande, et des moyens d'aspiration (26, 27) dudit air qui peuvent être activés par ladite unité de commande.

11. Système selon la revendication 1, ***caractérisé par le fait qu'**il* comprend des moyens (28) de chauffage et de refroidissement du sang contenu dans ledit circuit (3) de circulation induite.

12. Système selon la revendication 11, ***caractérisé par le fait que*** lesdits moyens de chauffage et de refroidissement comprennent un élément (28) chauffant et refroidissant qui est placé le long d'au moins un segment dudit circuit (3) de circulation induite.

13. Système selon la revendication 12, ***caractérisé par le fait que*** ledit élément (28) chauffant et refroidissant comprend au moins une cellule Peltier qui peut être raccordée à un circuit d'alimentation électrique.

14. Système selon la revendication 12 ou 13, ***caractérisé par le fait que*** ledit élément (28) chauffant et refroidissant comprend une pluralité de dites cellules Peltier placées les unes à côté des autres, avec au moins une feuille de métal placée entre ladite pluralité de cellules et ledit au moins un segment dudit circuit (3) de circulation induite.

15. Système selon la revendication 1 ou 2, ***caractérisé par le fait qu'**il* comprend des moyens de stockage de données physiologiques du patient et des événements monitorés par ladite unité de commande.

16. Système selon l'une ou plusieurs des revendications précédentes, ***caractérisé par de*** ***fait que*** lesdits moyens de stockage comprennent une carte mémoire.

17. Système selon la revendication 1 ou 2, ***caractérisé par le fait qu'**il* comprend au moins une imprimante intégrée qui peut être commandée par ladite unité de commande.

18. Système selon la revendication 1 ou 2, ***caractérisé par le fait qu'**il* comprend une seringue de type pompe seringue qui peut être contrôlée par ladite unité de commande pour l'administration contrôlée desdits fluides médicaux.

19. Système selon la revendication 1, ***caractérisé par le fait que*** ladite structure de support comprend un corps de boîtier (21) qui est supporté par un support (22) équipé de moyens de réglage des dimensions.

20. Système selon la revendication 19, ***caractérisé par le fait que*** lesdits moyens de réglage des dimensions comprennent un chariot qui est solidairement associé audit corps de boîtier et est guidé, par coulissement, sur un support vertical (23) qui part d'une base (24).

21. Système selon l'une quelconque des revendications 1, 7, 19, ***caractérisé par le fait qu***'il comprend un écran (20) relié à ladite unité de commande, qui est activé par contact et peut être replié sur ledit corps de boîtier (21) dans une position fermée.
